# EUROPEAN PATENT APPLICATION

(11) **EP 1 167 341 A1**
(43) Date of publication of application: **02.01.2002**
(21) Application number: 01305373.1
(22) Date of filing: 21.06.2001
(51) Int. Cl.: C07C 205/45, C07C 205/46, C07C 205/48, C07D 211/60, C07D 211/32, C07D 265/30, C07D 207/08, C07D 295/10, C07D 217/02, A61K 31/06, A61K 31/12, A61K 31/33

(54) **Substituted nitrated catechols, their use in the treatment of some central and peripheral nervous system disorders and pharmaceutical compositions containing them**

(30) Priority: 21.06.2000 GB 0015228
(71) Applicant: Portela & Ca., S.A., 4745-457 S. Mamede do Coronado (PT)
(72) Inventor: Learmonth, David Alexander, 4470 Maia (PT); Soares da Silva, Patricio Manuel Vieira Araujo, 4150 Porto (PT)
(74) Representative: Curtis, Philip Anthony

(57) **Abstract**

New compounds of formula I are described:

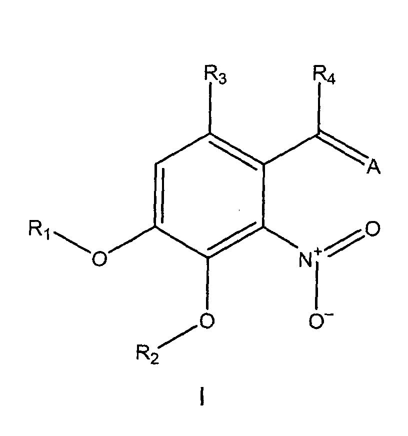

The compounds have potentially valuable pharmaceutical properties in the treatment of some central and peripheral nervous system disorders.

## Description

In recent years, the development of new inhibitors of the enzyme catechol-O-methyl transferase (COMT) has been accelerated by the hypothesis that inhibition of this enzyme may provide significant clinical improvements in patients afflicted by Parkinson's disease undergoing treatment with L-DOPA plus a peripheral AADC inhibitor. The rationale for the use of COMT inhibitors is based on their capacity to inhibit the *O*-methylation of L-DOPA to 3-*O*-methyl-L-Dopa. COMT inhibition slows elimination of L-DOPA from the plasma by increasing plasma half-life (increases area under the curve [AUC] without altering the time L-DOPA plasma to peak or the maximum concentration). Thus pharmacokinetic alterations may be an advantage over increasing the dose of L-DOPA, which also increases AUC, but additionally raises peak concentrations. With repeated doses of L-DOPA every 2-6 h in the presence of COMT inhibition, the mean plasma L-DOPA concentration is raised and the through concentrations are increased proportionally more than the peak concentrations despite a reduction in L-DOPA dose. As would be predicted by the slowed elimination of L-DOPA, the duration of antiparkinsonian action with single doses of L-DOPA is prolonged by COMT inhibition (Nutt, J.G., Lancet, 351:1221-1222, 1998). The most potent COMT inhibitors thusfar reported, 3,4-dihydroxy-4'-methyl-5-nitrobenzophenone (tolcapone, Australian Pat. AU-B-69764/87), and (E)-2-cyano-N,N-diethyl-3-(3,4-dihydroxy-5-nitrophenyl)acrylamide (entacapone, German Pat. DE 3740383 A 1) have inhibition constants in the low nM range. Tolcapone differs from entacapone in being a more potent inhibitor of COMT in the periphery and furthermore at penetrating into the brain to inhibit brain COMT as well. Due to unacceptable liver toxicity, only entacapone is currently used for the treatment of patients afflicted with Parkinson's disease undergoing treatment with L-DOPA plus a peripheral AADC inhibitor. Although the usefulness of inhibition of brain COMT has not been established for the treatment of Parkinson's disease (Hauser, R.A., et al., Mov Disord, 1998, 13, 643-647), there is evidence that COMT inhibitors penetrating the blood-brain barrier may be of interest in the treatment of mood disorders (Moreau, J.L., et al., Behav. Pharmacol., 1994, 5, 344-350; Fava, M. et al. J Clin Psychopharmacol, 1999, 19, 329-335).

As mentioned above, tolcapone and entacapone share the same pharmacophore, the 3,4-dihydroxy-5-nitrophenyl group. It is well established, that conjugation to either a carbonyl group as in tolcapone, or to a carbon-carbon double bond as in entacapone, greatly enhances inhibition of COMT. On the other hand, regioisomers of the pharmacophore, conjugated to formyl group were found only slightly active, with IC₅₀ in millimolar concentration range and approximately one order of magnitude more active when conjugated to carbon-carbon double bond forming analogous (3,4-dihydroxy-2-nitrophenyl)vinyl derivatives (R. A. Perez et al., Biochemical Pharmacology 45(10), 1973-1981(1993); R. A. Perez et al., J. Med. Chem. 35,4584-4588 (1992)). PCT WO 96/37456 discloses saturated analogues of the above mentioned vinyl derivatives, that are additionally substituted in position 6 by an electronegative substituent. These compounds are reported to have IC₅₀ in high nanomolar range.

We have found that incorporation of a substituted electron-withdrawing moiety to position 1 of the 3,4-dihydroxy-2-nitrophenyl group pharmacophore brings pronounced effects of potential usefulness for COMT inhibition. Conversely, compounds with the 3,4-dihydroxy-6-nitrophenyl substitution pattern possess very low COMT inhibitory activity. Similarly, only 2-nitro substituted compounds with free hydroxyl groups or the alkanoyl/aroyl protected derivatives which are hydrolysable under physiological conditions possess high and selective COMT inhibitory activity although from a synthetic viewpoint these compounds are not particularly readily accessible. The chemical literature does not reveal compounds possessing the requisite combination of free or alkanoyl/aroyl substituted 3,4-hydroxy groups adjacent to the 2-nitro group incorporating the substituted electron-withdrawing moiety necessary for highly potent and selective COMT inhibition, and all other compounds cited are always claimed for uses other than as COMT inhibitors. For example, GB 2088874 A (Example 20) claiming unrelated substituted quinazolines for the treatment of hypertension describes the preparation of 4-ethoxy-3-methoxy-2-nitroacetophenone as an intermediate (which contains two alkyloxy groups), though experimental evidence (NMR spectroscopy) clearly shows that the main product of nitration is in fact the regioisomer, 4-ethoxy-3-methoxy-6-nitroacetophenone (this compound was subsequently used in Example 21 of the same application). PCT WO99/32449 A1, claiming unrelated benzazine derivatives as phosphodiesterase 4 inhibitors describes in Example 30 a substituted 3-cyclopentyloxy-4-methoxy-2-nitrophenyl intermediate which due to the nature of both hydroxyl substituents (alkyloxy and cycloalkyloxy) could not be used as a COMT inhibitor. PCT WO 99/25335 A1 claims compounds based on the well-known aldol products derived from various substituted indanones and tetralones as inhibitors of cell proliferation, none of which however contain the required substitution pattern for COMT inhibition (e.g. p.26, lines 25-28 reveal a 5,6-dimethoxy-7-nitro compound, the alkoxy groups of which would completely prevent COMT inhibition). This group later published further structure-activity details on these compounds (H. Shih et al., Bioorganic and Medicinal Chemistry Letters, 10, 487-490 (2000)). Older reports such as in Bhakuni, D.S., et al., Indian J. Chem., Sect. B 24B(6), 596-601(1985); Chemical Abstracts 104:69049 referring to the synthesis of unrelated alkaloid compounds describe as an intermediate compound, the diazo-acetophenone (RN 99613-06-6) which contains alkyl and benzyl groups on the oxygen atoms and would be therefore unsuitable for COMT inhibition as well as undesirable. Chemical Abstracts 89:101857 and JP 53012421 A2 claims include some substituted alkyloxy nitro-indanones which again could not be used as COMT inhibitors.

The invention relates to compounds of formula I 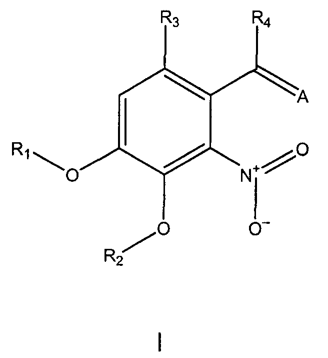 where R₁ and R₂ are the same or different and signify hydrogens or groups hydrolysable under physiological conditions, optionally substituted lower alkanoyl or aroyl, optionally substituted lower alkyl or arylsulphonyl or optionally substituted lower alkoxycarbonyl or optionally substituted lower alkylcarbamoyl, or taken together signify a lower alkylidene or cycloalkylidene group; R₃ signifies hydrogen or optionally substituted alkanoyl or aroyl group; R₄ signifies optionally substituted saturated or partially unsaturated lower alkyl or aryl group, or taken together with R₃ signifies an optionally substituted saturated or partially unsaturated carbocyclic ring; A signifies oxygen or NR₅ group, where R₅ signifies NHR₆ and R₆ signifies optionally substituted lower alkyl or aryl group, or OR₇ group where R₇ signifies hydrogen, lower alkyl or lower alkanoyl, or A signifies an optionally substituted alkylidene when R₄ signifies OR₈ group where R₈ signifies optionally substituted lower alkanoyl or aroyl group, and pharmaceutically acceptable salts thereof.

The term "lower" denotes residues with a maximum of 8, preferentially with a maximum of 4 carbon atoms. The term "alkyl" taken alone or in combination with terms such as "alkanoyl, alkoxycarbonyl, alkylidene, cycloalkylidene, alkoxycarbonyloxy, alkylamino " denotes straight-chain or branched saturated hydrocarbon residues. The term halogen denotes fluorine, chlorine, bromine, and iodine. The term "aryl" denotes a optionally substituted carbocyclic aromatic group, preferably mono- or bicyclic groups.

For the preparation of pharmaceutical compositions of compounds of formula I, inert pharmaceutically acceptable carriers are admixed with the active compounds. The pharmaceutically acceptable carriers may be either solid or liquid. Solid form preparations include powders, tablets, dispersible granules and capsules. A solid carrier can be one or more substances which may also act as diluents, flavouring agents, solubilizers, lubricants, suspending agents, binders or tablet disintegrating agents; it may also be an encapsulating material.

Preferably, the pharmaceutical preparation is in unit dosage form, e.g. packaged preparation, the package containing discrete quantities of preparation such as packeted tablets, capsules and powders in vials or ampoules.

The dosages may be varied depending on the requirement of the patient, the severity of the disease and the particular compound being employed. For convenience, the total daily dosage may be divided and administered in portions throughout the day. Determination of the proper dosage for a particular situation is within the skill of those in the medical art.

### Material and Methods

### Studies in rat tissues

Tissues (liver and brain) from 60 days old male Wistar rats weighing 200-250 g (Harlan-Interfauna Ibérica, Barcelona, Spain) kept two per cage under controlled environmental conditions (12 h light/dark cycle and room temperature 24 °C), were used in all experiments. Saline perfused tissues, obtained from pentobarbitone (60 mg/Kg) anaesthetised rats, were used in the experiments. Tissues were immediately removed and homogenised in 5 mM phosphate buffer, pH 7.8 and stored at -80°C.

COMT activity was evaluated by the ability to methylate adrenaline to metanephrine, as previously described (Vieira-Coelho, M.A., Soares-da-Silva, P., Brain Res, 1999, 821,69-78). Aliquots of 0.5 ml of liver and brain homogenates were preincubated for 20 min with 0.4 ml of phosphate buffer (5 mM); thereafter, the reaction mixture was incubated for 5 min (liver) or 15 min (brain) with increasing concentrations of adrenaline (0.1 to 2000 µM; 0.1 ml) in the presence of a saturating concentration of S-adenosyl-L-methionine, the methyl donor (liver, 500 µM; brain, 100 µM). The incubation medium also contained pargyline (100 µM), MgCl₂ (100 µM) and EGTA (1 mM). The preincubation and incubation were carried out at 37°C under conditions of light protection with continuous shaking and without oxygenation.

In experiments conducted with the aim of studying the inhibitory effect of COMT inhibitors on enzyme activity, the reaction mixture was preincubated for 20 min with increasing concentrations of test compounds (0.1 to 3,000 nM); the incubation was performed in the presence of a concentration of adrenaline five times the corresponding Kₘ value as determined in saturation experiments. At the end of the incubation period the tubes were transferred to ice and the reaction was stopped by the addition of 200 µl of 2 M perchloric acid. The samples were then centrifuged (200×*g*, 4 min, 4°C), and 500 µl aliquots of the supernatant, filtered on 0.22 µm pore size Spin-X filter tubes (Costar) were used for the assay of metanephrine.

In experiments designed to evaluate the oral bioavailability, half-life and brain access, test compounds (in saline with 10 % tween 80) were given by gastric tube to overnight fasted rats. Thereafter, at defined intervals, livers and brains were removed and used to determine COMT activity as described above.

### Studies in Human neuroblastoma cells

SK-N-SH cells (ATCC HTB-11) were obtained from the American Type Culture Collection and maintained in a humidified atmosphere of 5% CO₂-95% air at 37°C. The cells were grown in Minimal Essential Medium supplemented with 10% foetal bovine serum, 100 U/ml penicillin G, 0.25 µg/ml amphotericin B, 100 µg/ml streptomycin, and 25 mM *N*-2-hydroxyethylpiperazine-*N*'-2-ethanosulfonic acid (HEPES). For subculturing, the cells were dissociated with 0.05% trypsin-EDTA, split 1:4 and subcultured in Costar flasks with 21-cm² growth areas (Costar, Badhoevedorp, The Netherlands). For *O*-methylation studies, the cells were seeded in 96 well plates and 24 hours prior to each experiment the medium was changed to medium free of foetal bovine serum. The cell medium was changed every two days and experiments were generally performed after cells reached confluence (5-7 days) and each cm² contained about 100 µg of cell protein. On the day of the experiment, the growth medium was aspirated and the cells washed with phosphate buffer (5 mM). COMT activity was evaluated in cell monolayers by the ability to methylate adrenaline (0.03 to 100 µM) to metanephrine in the presence of saturating concentration of the S-adenosyl-L-methionine, the methyl donor (250 µM), pargyline (100 µM), MgCl₂ (100 µM) and EGTA (1 mM). The preincubation and incubation were carried out at 37°C under conditions of light protection with continuous shaking and without oxygenation. After preincubation, cells were incubated for 15 min with 1000 µM epinephrine. The reaction was terminated by the addition of 15 µl of 2 M perchloric acid. The acidified samples were stored at 4°C before injection into the high pressure liquid chromatograph for the assay of metanephrine. The assay of metanephrine was carried out by means of high pressure liquid chromatography with electrochemical detection. The lower limits for detection of metanephrine ranged from 350 to 500 fmol (0.5 to 1.0 pmol/mg protein/h).

Kₘ and Vₘₐₓ values for COMT activity were calculated from non-linear regression analysis using the GraphPad Prism statistics software package (Motulsky, H.G., et al., GraphPad Prisms, GraphPad Prism Software Inc., San Diego, 1994). For the calculation of the IC₅₀ values, the parameters of the equation for one site inhibition were fitted to the experimental data. Geometric means are given with 95% confidence limits and arithmetic means are given with S.E.M.. Statistical analysis was performed by one-way analysis of variance (ANOVA) using Newman-Keuls multiple comparison test to compare values.

The protein content in the homogenates was determined by the method of Bradford (Bradford, M.M., Anal. Biochem., 72: 248-254, 1976) with human serum albumin as standard. The protein content was similar in all samples (approximately 2 mg/500 µl homogenate).

### Results

### In vitro COMT inhibition studies

Incubation of liver and whole brain homogenates in the presence of increasing concentrations of adrenaline resulted in a concentration-dependent formation of metanephrine, yielding Kₘ (in µM) and Vₘₐₓ (in nmol mg protein⁻¹ h⁻¹) values of 0.7 (0.5, 0.9; 95% confidence intervals) and 1.31±0.02 for brain and 238.5 (128.5; 348.5) and 61.6±3.8 for liver, respectively. From these kinetic parameters, a saturating concentration of adrenaline was chosen to use in inhibition studies (liver, adrenaline = 1000 µM; brain, adrenaline = 100 µM).

Compounds of formulae 1 - 51 and entacapone (the reference compound) produced marked decreases in the *O*-methylation of adrenaline in both the liver and brain homogenates (see table 1). When tested for inhibition of COMT in intact SK-N-SH cells, the listed compounds were also found to markedly reduced the *O*-methylation of adrenaline (see table 1).

### In vitro ex vivo COMT inhibition studies

Compounds **20** and **24** were found to be potent inhibitors of brain COMT, the maximal inhibitory effect being achieved within 30 min after their oral administration (table 2). Compound **24** presented a similar inhibitory profile in brain and liver COMT, whereas compound **20** was much more potent upon brain COMT than liver COMT. Similarly, compound **5** was also much more potent as a peripheral COMT inhibitor than in brain, as observed with entacapone.

**Table 1.**

| Effect of compounds 1 - 51 and entacapone upon COMT activity in homogenates of rat liver and brain and in SK-N-SH cells. The concentration of test compound was 3 µM in liver homogenates and 100 nM in brain homogenates and SK-N-SH cells. Results are means ±SEM of n=4-8. | | | |
|---|---|---|---|
| Compounds | Liver (% of control) | Brain (% of control) | SK-N-SH (% of control) |
| 1 | 1.2±0.2 | 15±4 | 46±1 |
| 2 | 1.3±0.7 | 9±1 | 24±1 |
| 3 | 5.2±0.0 | 85±2 | 94±1 |
| 4 | 2.0±1.0 | 20±2 | 25±1 |
| 5 | 0.3±0.1 | 6±1 | 2±0 |
| 6 | 1.77±0.1 | 33±1 | 39±3 |
| 7 | 0.6±0.3 | 14±1 | 23±1 |
| 8 | 1.0±0.1 | 30±4 | 48±3 |
| 9 | 9.0±1.6 | 47±5 | 8±1 |
| 10 | 0.3±0.1 | 14±1 | 24±2 |
| 11 | 1.1±0.3 | 32±2 | 47±2 |
| 12 | 0.6±0.0 | 18±1 | 34±1 |
| 13 | 2.3±0.1 | 27±1 | 46±1 |
| 14 | 0.3±0.0 | 5±1 | 5±2 |
| 15 | 0.5±0.0 | 7±2 | 13±4 |
| 16 | 2.0±0.2 | 20±1 | 92±1 |
| 17 | 0.4±0.0 | 8±1 | 24±4 |
| 18 | 2.0±0.0 | 17±1 | 70±5 |
| 19 | 0.2±0.1 | 8±1 | 0±0 |
| 20 | 0.6±0.0 | 3±0 | 0±0 |
| 21 | 0.3±0.2 | 11±1 | 1±1 |
| 22 | 0.3±0.1 | 9±0 | 12±1 |
| 23 | 2.5±1.2 | 10±1 | 24±1 |
| 24 | 0.7±0.0 | 7±1 | 24±1 |
| 25 | 1.7±0.1 | 19±1 | 39±1 |
| 26 | 0.9±0.1 | 9±0 | 12±1 |
| 27 | 2.4±0.2 | 15±1 | 5±1 |
| 28 | 2.2±0.2 | 14±1 | 7±0 |
| 29 | 0.6±0.0 | 12±1 | 21±1 |
| 30 | 1.4±0.1 | 8±0 | 17±1 |
| 31 | 1.7±0.6 | 8±0 | 13±2 |
| 32 | 2.3±0.2 | 10±0 | 11±2 |
| 33 | 2.2±0.3 | 6±0 | 14±0 |
| 34 | 1.6±0.2 | 5±0 | 11±2 |
| 35 | 23.±0.3 | 9±0 | 13±1 |
| 36 | 2.2±0.4 | 6±0 | 9±2 |
| 37 | 1.7±0.3 | 6±0 | 6±2 |
| 38 | 11.2±1.5 | 7±0 | 6±1 |
| 39 | 0.8±0.2 | 8±0 | 11±0 |
| 40 | 1.6±0.4 | 10±0 | 8±1 |
| 41 | 1.1±0.2 | 9±1 | 5±0 |
| 42 | 0.8±0.2 | 13±0 | 27±1 |
| 43 | 0.9±0.2 | 10±1 | 19±7 |
| 44 | 0.6±0.2 | 10±0 | 9±2 |
| 45 | 0.7±0.2 | 0±0 | 7±3 |
| 46 | 0.7±0.2 | 0±0 | 1±1 |
| 47 | 0.6±0.2 | 0±0 | 1±1 |
| 48 | 0.5±0:1 | 0±0 | 7±3 |
| 49 | 1.4±0.8 | 0±0 | 1±0 |
| 50 | 0.6±0.5 | 0±0 | 2±1 |
| 51 | 0.6±0.3 | 0±0 | 1±0 |
| Entacapone | 0.7±0.2 | 8±1 | 23±3 |

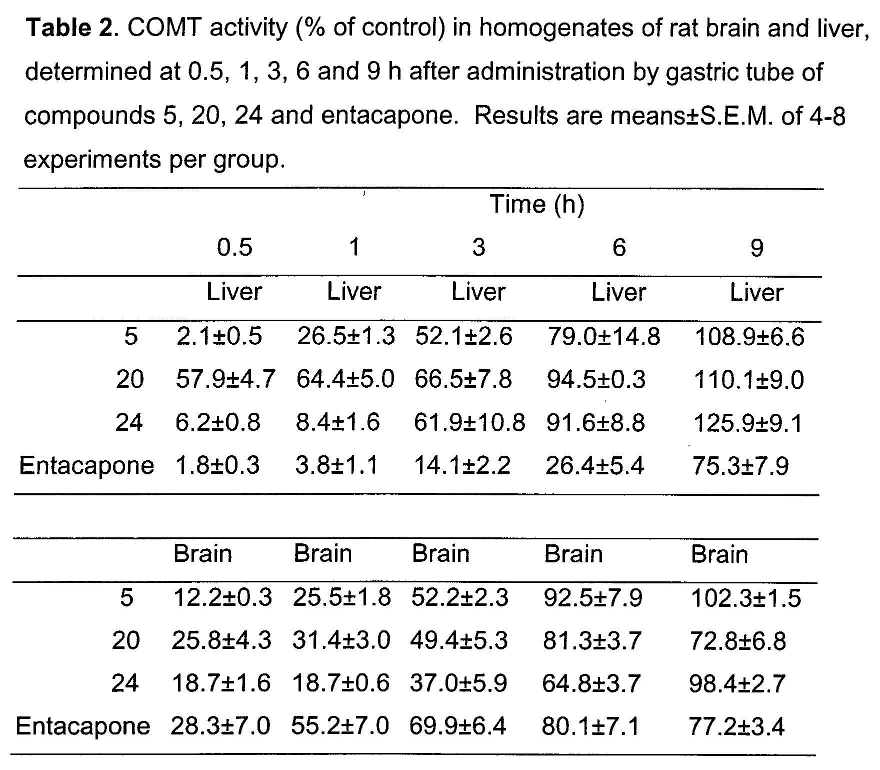

### Conclusion

Compounds of formula I are very potent catechol-*O*-methyltransferase (COMT) inhibitors and have potentially valuable pharmaceutical properties in the treatment of some central and peripheral nervous system disorders where inhibition of *O*-methylation of catecholamines may be of therapeutical benefit, such as mood disorders, Parkinson' disease and parkinsonian disorders, gastrointestinal disturbances, edema formation states and hypertension. The possibility to use a COMT inhibitor with enhanced access to the brain and limited activity in the periphery, such as compound **20**, opens new perspectives in the therapy of mood disorders by improving selectivity on COMT inhibition. On the other hand, the use of COMT inhibitor with limited access to the brain, such as compounds **5** and **24**, opens new perspectives in the therapy of Parkinson' disease and parkinsonian disorders, gastrointestinal disturbances, edema formation states and hypertension, by improving selectivity of COMT inhibition in the periphery. This is particularly important when thinking of treating patients afflicted by Parkinson's disease and taking L-DOPA plus a peripheral AADC inhibitor, due to the possibility that COMT inhibitors which have easy access to the brain may cause excessive dopaminergic stimulation, namely by inducing dyskinesia and mental confusion in L-DOPA treated patients.

The invention disclosed here within is exemplified by the following examples of preparation, which should not be construed to limit the scope of the disclosure. Alternative pathways and analogous structures may be apparent to those skilled in the art.

### Example 1

### 1-(3,4-dihydroxy-2-nitro-phenyl)-2-morpholin-4-yl-ethanone (compound 42, Table 1)

A solution of 3,4-dihydroxy-2-nitroacetophenone (3.0 g, 15.20 mmol) in tetrahydofuran (30 mL) was stirred at room temperature, phenyltrimethylammonium tribromide (6.90 g, 19 mmol) was added, and the reaction mixture was stirred for twenty minutes. The precipitate was filtered off and the organic phase was diluted with water (100 mL) and extracted by dichloromethane (2 x 100 mL). The organic extract was washed with brine, dried over sodium sulphate, and the volatile components were evaporated under vacuum, leaving a yellow oil that was dissolved in hot toluene. After standing for 16 hours at room temperature, the formed crystals were filtered off, washed with cold toluene and dried to constant weight in vacuo.

The alpha-bromoketone from above (1.38 g, 5 mmol) was dissolved in acetonitrile (14 mL) and morpholine (1.5 mL, 15 mmol) was added. The reaction mixture was stirred for 2 hours at room temperature whereupon an excess of HCI in ethanol was added, and the precipitate formed was filtered off, washed by cold absolute ethanol and dried under vacuum to constant weight to give yellow crystals, m.p. 209 to 212° C.

### Examples 2-3

By the application of the above described technique and related procedures known to those skilled in the art, and using the appropriate secondary amines, the following compounds were prepared:
1 -(3,4-Dihydroxy-2-nitro-phenyl)-2-pyrrolidin-1-yl-ethanone
1-(3,4-Dihydroxy-2-nitro-phenyl)-2-piperidin-1-yl-ethanone

### Example 4

### Carbonic acid 4,5-dibenzoyl-2-ethoxycarbonyloxy-3-nitro-phenyl ester ethyl ester (compound 23, Table 1)

(2-Benzoyl-4,5-dimethoxy-phenyl)-phenyl-methanone (1.45 g, 4.19 mmol) and pyridinium chloride (2.31 g, 20 mmol) were heated at 220° C for 2 hours, and the resulting dark reaction mixture was poured onto ice/water. Precipitated (2-benzoyl-4,5-dihydroxy-phenyl)-phenyl-methanone was filtered off, dried and used as such for the next step.

The substituted catechol from above (0.36 g, 1 mmol) was dissolved in dichloromethane (20 mL), and the cooled (-10° C) solution was treated with mmol 100% nitric acid (1.1 mmol). The reaction mixture was washed with brine, dried over anhydrous sodium sulphate, and filtered. Pyridine (0.24 g, 3 mmol) and a catalytic amount of 4-dimethyaminopyridine were added to the solution, followed by ethylchloroformate (0.32 g, 3 mmol). The reaction mixture was stirred at room temperature for 2 hours and then washed with a cold 0.1 M solution of phosphoric acid and brine, then dried over sodium sulphate, filtered and the volatile components were evaporated under vacuum, leaving a white precipitate, which was recrystallized from a mixture of dichloromethane and petroleum ether to give white crystals, m.p. 164 to 166°C.

### Example 5

### 5-Benzyloxy-6-hydroxy-indan-1-one

To a stirred solution of 5,6-dihydroxy-indan-1-one (5.23 g. 31.91 mmol) in dimethylformamide (140 mL) at room temperature was added potassium carbonate (4.40 g, 31.80 mmol) followed by benzyl bromide (5.44 g, 31.80 mmol) and the resulting suspension was stirred at 90 °C for three hours. After cooling to room temperature, the inorganic material was filtered off and the filter cake was washed by dimethylformamide (10 mL). The combined filtrate was then evaporated in vacuo and the residue was treated with water (50 mL). The precipitated solid was removed by filtration and recrystallised from ethanol to give pale brown crystals of m.p. 140 to 142 °C.

### Example 6

### 5-Benzyloxy-6-hydroxy-7-nitro-indan-1-one

To a stirred and cooled (water bath) suspension of 5-benzyloxy-6-hydroxy-indan-1-one (4.71 g, 18.55 mmol) in glacial acetic acid (50 mL) was added dropwise 65% nitric acid (5.1 mL, 72.42 mmol). The mixture became initially deep red in appearance followed by the formation of a new orange precipitate. After stirring for 1.5 hours at room temperature, the mixture was poured onto ice-water (150 mL) and the precipitate was filtered off. The solid was triturated with warm methanol (30 mL) and filtered while still warm to give orange crystals of m.p. 252 to 254 °C.

### Example 7

### 5,6-Dihydroxy-7-nitro-indan-1-one (compound 2, Table 1)

A stirred suspension of 5-benzyloxy-6-hydroxy-7-nitro-indan-1-one (3.57 g, 11.94 mmol) in a mixture of 48% aqueous hydrobromic acid (35 mL) and 30% hydrogen bromide in acetic acid (35 mL) was heated to 120 °C for fifteen minutes and then allowed to cool to room temperature. The resulting dark mixture was poured onto ice-water (250 mL) and the brown precipitate was filtered off. The filtrate was extracted by ethyl acetate (50 mL) and the organic phase was washed by water and brine, then dried over sodium sulphate, filtered and evaporated in vacuo to leave an orange solid which was combined with the above precipitate and recrystallised from ethyl acetate to give yellow crystals which decomposed without melting above 230°C.

### Examples 8-9

By the application of the above described technique and related procedures known to those skilled on the art and using the appropriate benzyl ethers, the following compounds were prepared:
6,7-Dihydroxy-8-nitro-3,4-dihydro-2*H*-naphthalen-1-one (compound 1, Table 1)
2,3-Dihydroxy-4-nitro-6,7,8,9-tetrahydro-benzocyclohepten-5-one

### Example 10

### 5,6-Dihydroxy-7-nitro-indan-1-one oxime (compound 6, Table 1)

A stirred solution of 5,6-dihydroxy-7-nitro-indan-1-one (0.1 g, 0.48 mmol) in ethanol (5 mL) at room temperature was treated with pyridine (0.13 g, 1.67 mmol) and hydroxylamine hydrochloride (0.11 g, 1.53 mmol). The resulting mixture was heated at 85 °C for three hours and then allowed to cool to room temperature. The solvent was removed in vacuo to leave a red oil which was partitioned between ethyl acetate (5 mL) and water (5 mL). The organic phase was separated and washed by 1N HCI, water and brine, then dried over sodium sulphate, filtered and evaporated in vacuo. The residue was recrystallised from water to give reddish needles of m.p. 200 to 201 °C.

### Examples 11-13

By the application of the above described technique and related procedures known to those skilled in the art and using the appropriate bicyclic ketones, the following compounds were prepared:
6,7-Dihydroxy-8-nitro-3,4-dihydro-2*H*-naphthalen-1-one oxime
1-[(2,4-dinitrophenyl)-hydazono]-7-nitro-indan-5,6-diol
8-[(2,4-Dinitrophenyl)-hydrazono]-1-nitro-5,6,7,8-tetrahydro-naphthalen-2,3-diol

### Example 14

### 2-(3,4-Dimethoxy-benzylidene)-5,6-dihydroxy-7-nitro-indan-1-one (compound 7, Table 1)

A solution of 5,6-dihydroxy-7-nitro-indan-1-one (0.10 g, 0.48 mmol) and 3,4-dimethoxybenzaldehyde (0.095 g, 0.57 mmol) in methanol (5 mL) was stirred with ice-bath cooling whilst dry hydrogen chloride gas was passed through the reaction mixture. Quickly an exothermic reaction started which was accompanied by a colour change to deep red and after ten minutes the reaction mixture was poured onto ice-water (20 mL). The precipitated solid was removed by filtration and triturated with warm methanol (5 mL) to give yellow crystals which decomposed without melting above 265 °C.

### Examples 15-23

By the application of the above described technique and related procedures known to those skilled in the art and using the appropriately substituted aldehydes, the following compounds were prepared:
2-(3,4-Dimethoxy-benzylidene)-6,7-dihydroxy-8-nitro-3,4-dihydro-2*H*-naphthalen-1-one (compound 4, Table 1)
2-(4-Dimethylamino-benzylidene)-6,7-dihydroxy-8-nitro-3,4-dihydro-2*H*-naphthalen-1-one (compound 9, Table 1)
5,6-Dihydroxy-2-(4-hydroxy-3-methoxy-5-nitro-benzylidene)-7-nitro-indan-1-one (compound 10, Table 1)
6,7-Dihydroxy-2-(4-hydroxy-3-methoxy-5-nitro-benzylidene)-8-nitro-3,4-dihydro-2*H*-naphthalen-1-one (compound 11, Table 1)
2-(3,4-dihydroxy-benzylidene)-5,6-dihydroxy-7-nitro-indan-1-one (compound 12, Table 1)
2-(3,4-Dihydroxy-benzylidene)-6,7-dihydroxy-8-nitro-3,4-dihydro-2H-naphthalen-1-one (compound 13, Table 1)
2-(4-dimethylamino-benzylidene)-5,6-dihydroxy-7-nitro-indan-1-one (compound 8, Table 1)
2-(3,4-Dihydroxy-5-nitro-benzylidene)-5,6-dihydroxy-7-nitro-indan-1-one (compound 16, Table 1)
1-(3,4-dihydroxy-2-nitro-phenyl)-3-phenyl-propenone (compound 41, Table 1)

### Example 24

### 2-Hydroxy-3-Methoxy-1-nitro-anthraquinone

A suspension of 2-hydroxy-3-methoxy-anthraquinone (2.91 g, 11.45 mmol) in dichloromethane (100 mL) was stirred with ice-bath cooling whilst concentrated nitric acid (2.36 mL, 57.26 mmol) was added dropwise. During addition of the acid, the original flocculent orange solid turned to a fine light brown precipitate. After stirring at room temperature for twenty minutes, the mixture was poured onto ice-water (350 mL) and the precipitate was removed by filtration and recrystallised from a dichloromethane/ethanol mixture to give yellow crystals of m.p. 296 to 297 °C.

### Example 25

### 2,3-Dihydroxy-1-nitro-anthraquinone (compound 18, Table 1)

A stirred suspension of 2-hydroxy-3-methoxy-1-nitro-anthraquinone (2.20 g, 7.36 mmol) in 1,1,2,2-tetrachloroethane (90 mL) at room temperature was treated with aluminium chloride (1.75 g, 13.16 mmol) in one portion followed by the dropwise addition of pyridine (2.56 g, 32.39 mmol). The resulting mixture was stirred at 95 °C for two hours and then allowed to cool to room temperature and quenched by the addition of 2N HCI. The precipitated solid was removed by filtration and recrystallised from acetic acid to afford orange crystals which decomposed without melting above 256 °C.

### Example 26

### 5,6-Dihydroxy-2-morpholin-4-ylmethyl-7-nitro-indan-1-one (compound 29, Table 1)

To a stirred suspension of 5,6-dihydroxy-7-nitro-indan-1-one (0.091 g, 0.43 mmol) in ispropanol (4 mL) at room temperature was added morpholine (0.15 g, 1.73 mmol) followed by 35% aqueous formaldehyde solution (0.17 mL, 2.17 mmol) and concentrated hydrochloric acid (0.21 mL, 2.60 mmol). The resulting mixture was heated at reflux for three hours during which time a precipitate formed. After cooling to room temperature, the precipitate was removed by filtration and washed by isopropanol (1 mL) to afford off-white crystals of m.p. 193 to 195 °C.

### Examples 27-41

By the application of the above described technique and related procedures known to those skilled in the art and using appropriate secondary amines, the following compounds were prepared:
5,6-Dihydroxy-7-nitro-2-piperidin-1-ylmethyl-indan-1-one (compound 30, Table 1)
5,6-Dihydroxy-7-nitro-2-[4-(3-trifluoromethyl-phenyl)-piperazin-1-ylmethyl]-indan-1-one (compound 43, Table 1)
5,6-Dihydroxy-7-nitro-2-(4-phenyl-piperazin-1-ylmethyl)-indan-1-one (compound 44, Table 1)
1-(3,4-dihydroxy-2-nitro-phenyl)-3-morpholin-4-yl-propan-1-one (compound 26, Table 1)
1-(3,4-Dihydroxy-2-nitro-phenyl)-3-piperidin-1-yl-propan-1-one (compound 27, Table 1)
1-(3,4-Dihydroxy-2-nitro-phenyl)-3-pyrrolidin-1-yl-propan-1-one (compound 28, Table 1)
1-[3-(3,4-Dihydroxy-2-nitro-phenyl)-3-oxo-propyl]-piperidine-3-carboxylic acid diethylamide (compound 31, Table 1)
1-(3,4-Dihydroxy-2-nitro-phenyl)-3-(3-methyl-piperidin-1-yl)-propan-1-one (compound 32, Table 1)
1-(3,4-Dihydroxy-2-nitro-phenyl)-3-(4-methyl-piperidin-1-yl)-propan-1-one (compound 33, Table 1)
1-(3,4-Dihydroxy-2-nitro-phenyl)-3-(octahydro-quinolin-1 -yl)-propan-1-one (compound 34, Table 1)
1-(3,4-Dihydroxy-2-nitro-phenyl)-3-(3,5-dimethyl-piperidin-1-yl)-propan-1-one (compound 35, Table 1)
3-(4-benzyl-piperidin-1-yl)-1-(3,4-Dihydroxy-2-nitro-phenyl)-propan-1-one (compound 36, Table 1)
1-(3,4-Dihydroxy-2-nitro-phenyl)-3-[4-(4-methoxy-phenyl)-piperazin-1-yl]-propan-1-one (compound 37, Table 1)
1-(3,4-Dihydroxy-2-nitro-phenyl)-3-[4-(3-trifluoromethyl-phenyl)-piperazin-1-yl]-propan-1-one (compound 38, Table 1)
1-(3,4-Dihydroxy-2-nitro-phenyl)-3-(4-propyl-piperazin-1-yl)-propan-1-one (compound 39, Table 1)

### Example 42

### 3,4-Dihydro-6,7-dihydroxy-2,2-dimethyl-2H-naphthalen-1-one

A suspension of 3,4-dihydro-6,7-dimethoxy-2,2-dimethyl-1(2H)-naphthalenone (2.37 g, 10.10 mmol) in 48% hydrobromic acid (70 mL) was heated at 140 °C for three hours to give a deep brown solution which was allowed to cool to room temperature and then poured onto ice-water (300 mL). The resulting precipitate was filtered off and washed by water (20 mL). The filtrate was the extracted by ethyl acetate (100 mL) and the organic phase was washed by brine then dried over sodium sulphate, filtered and evaporated in vacuo to leave a brown solid which was combined with the precipitate from above and recrystallised from water to afford pale brown crystals of melting point 163 to 165 °C.

### Example 43

### 6-Benzyloxy-3,4-dihydro-7-hydroxy-2,3-dimethyl-2H-naphthalen-1-one

A stirred solution of 3,4-dihydro-6,7-dihydroxy-2,2-dimethyl-2H-naphthalen-1-one (1.34 g, 6.52 mmol) in dimethylformamide (30 mL) at room temperature was treated with potassium carbonate (0.90 g, 6.52 mmol) in one portion followed by benzyl bromide (1.12 g, 6.52 mmol). The resulting suspension was stirred at 80 °C for 3 hours and then allowed to cool to room temperature. The inorganic material was removed by filtration and the filter cake was washed by dimethylformamide (5 mL). The combined filtrate was evaporated in vacuo and the residue was partitioned between ethyl acetate (30 mL) and water (30 mL). The organic phase was separated and washed by brine, then dried over sodium sulphate, filtered and evaporated in vacuo. The residue was chromatographed over silica gel using a petroleum ether/ethyl acetate mixture to give the product as a yellow oil which crystallised on standing to a pale yellow solid of m.p. 90 to 93 °C.

### Example 44

### 6-Benzyloxy-3,4-dihydro-2,2-dimethyl-7-hydroxy-8-nitro-2H-naphthalen-1-one

To an almost solution of 6-benzyloxy-3,4-dihydro-7-dihydroxy-2,2-dimethyl-2H-naphthalen-1-one (1.34 g, 4.54 mmol) in glacial acetic acid (30 mL) cooled in a water bath was added dropwise with stirring 58% nitric acid (0.43 mL, 5.45 mmol). The resulting deep red solution was stirred at room temperature for fifteen minutes and then poured onto ice-water (100 mL). The resulting precipitate was filtered off and dissolved in dichloromethane (30 mL) then washed by water and brine and dried over sodium sulphate. Filtration and evaporation in vacuo afforded a brown oil which solidified on standing. Recrystallisation from a dichloromethane/diisopropyl ether mixture gave light brown crystals of m.p. 146 to 149 °C.

### Example 45

### Acetic acid, 3-acetoxy-7,7-dimethyl-1-nitro-8-oxo-5,6,7,8-tetrahydro-naphthalen-2-yl ester (compound 25, Table 1)

A stirred, yellow solution of 6-benzyloxy-3,4-dihydro-2,2-dimethyl-7-hydroxy-8-nitro-2H-naphthalen-1-one (0.70 g, 2.06 mmol) in dichloromethane (20 mL) cooled in an ice-water bath was treated dropwise with 30% hydrogen bromide in acetic acid (4.1 mL, 20.5 mmol). The resulting orange solution was stirred at room temperature for twenty-four hours and then poured onto ice-water (150 mL). The pale yellow precipitate was removed by filtration and dissolved in ethyl acetate (15 mL) and washed by brine, then dried over sodium sulphate. Filtration and evaporation in vacuo afforded a pale yellow solid which was suspended in dichloromethane (10 mL) and treated with pyridine (0.57 g, 7.26 mmol), acetic anhydride (0.74 g, 7.26 mmol) and 4-dimethylaminopyridine (0.01 g). After stirring at room temperature for thirty minutes, the resulting solution was washed with cold water, 1N HCI, water again and brine, then dried over sodium sulphate. The solvent was removed in vacuo and toluene (10 mL) was added to the residue. After re-evaporation, the residual pale yellow solid was recrystallised from a dichloromethane/ethanol mixture to give white crystals of m.p. 145 to 147 °C.

### Example 46

### Acetic acid, 2-acetoxy-4-(1-acetoxy-2-phenyl-vinyl)-3-nitro-phenyl ester (compound 46, Table 1)

To a stirred suspension of 1-(3,4-dihydroxy-2-nitro-phenyl)-2-phenylethanone (0.40 g, 1.46 mmol) in dichloromethane (6 mL) at room temperature was added pyridine (0.46 g, 5.86 mmol), acetic anhydride (0.6 g, 5.86 mmol) and 4-dimethylaminopyridine (0.01 g). The resulting solution was stirred for for one hour and then extracted with cold water, 1N HCI and brine, then dried over sodium sulphate. After filtration and evaporation in vacuo, the residue was recrystallised from a dichloromethane/ethanol mixture to afford off-white crystals of m.p. 119 to 121 °C.

### Example 47

By the application of the above described technique and related procedures known to those skilled in the art and using 5,6-dihydroxy-7-nitro-1-indanone, the following compound was prepared:
Acetic acid, 1,6-diacetoxy-7-nitro-3*H*-inden-5-yl ester

### Example 48

### Butyric acid, 3-benzoyl-6-butyryloxy-2-nitro-phenyl ester (compound 20, Table 1)

To a stirred solution of (3,4-dihydroxy-2-nitro-phenyl)-phenyl-methanone (0.34 g, 1.29 mmol) in dichloromethane (5 mL) at room temperature was added pyridine (0.41 g, 5.19 mmol), butyric anhydride (0.82 g, 5.19 mmol) and 4-dimethyl-aminopyridine (0.01 g). The resulting solution was stirred for one hour and then extracted by cold water, 1N HCI and brine, then dried over sodium sulphate. After filtration and evaporation in vacuo the residue was chromatographed over silica gel using an ethyl acetate/petroleum ether mixture to give off-white crystals of m.p 55 to 57 °C.

### Examples 49-58

By the application of the above described technique and related procedures known to those skilled in the art and using appropriate acylating reagents, the following compounds were prepared:
Carbonic acid, 4-benzoyl-2-ethoxycarbonyloxy-3-nitro-phenyl ester ethyl ester (compound 21, Table 1)
Butyric acid, 6-butyryloxy-2-nitro-3-(3-phenyl-propionyl)-phenyl ester (compound 19, Table 1)
Carbonic acid, 2-ethoxycarbonyloxy-3-nitro-4-(3-phenyl-propionyl)-phenyl ester ethyl ester (compound 22, Table 1)
Acetic acid, 6-acetoxy-2-nitro-3-(3-phenyl-acryloyl)-phenyl ester (compound 40, Table 1)
Acetic acid, 6-acetoxy-2-nitro-3-phenylacetyl-phenyl ester (compound 45, Table 1)
Butyric acid, 6-butyryloxy-2-nitro-3-phenylacetyl-phenyl ester (compound 47, Table 1)
Carbonic acid, 2-ethoxycarbonyloxy-3-nitro-4-phenylacetyl-phenyl ester, ethyl ester (compound 48, Table 1)
Acetic acid, 6-acetoxy-2-nitro-3-(4-phenyl-butyryl)-phenyl ester (compound 49, Table 1)
Acetic acid, 6-butyryloxy-2-nitro-3-(4-phenyl-butyryl)-phenyl ester (compound 50, Table 1)
Carbonic acid, 2-ethoxycarbonyloxy-3-nitro-4-(4-phenyl-butyryl)-phenyl ester ethyl ester (compound 51, Table 1)

### Example 59

### Acetic acid, 4-benzoyl-2-methoxy-3-nitro-phenyl ester

To a stirred solution of acetic acid, 4-benzoyl-2-methoxy phenyl ester (7.25 g, 26.9 mmol) in acetic anhydride (79 mL) at room temperature was added cupric nitrate trihydrate (8.37 g, 34.65 mmol) in one portion. After seven minutes an exothermic reaction set in which was controlled by ice-bath cooling. When exotherm had subsided, the reaction mixture was poured onto ice-water (200 mL) and the yellow precipitate was extracted by dichloromethane (150 mL). The organic phase was washed by water and brine, then dried over sodium sulphate, filtered and evaporated in vacuo . The residue was chromatographed over silica gel using a dichloromethane/petroleum ether mixture to give an off-white solid that was recrystallised from ethanol to afford white crystals of m.p. 86 to 87 °C.

### Example 60

### (4-Hydroxy-3-methoxy-2-nitro-phenyl)-phenyl-methanone

A stirred suspension of the the acetate from above (4.28 g, 13.60 mmol) in methanol (70 mL) at room temperature was treated with 3N aqueous sodium hydroxide solution (13.6 mL, 40.8 mmol) and the resulting orange solution was stirred for twenty minutes and then poured onto water (200 mL). The aqueous phase was then acidified with 2N HCI and extracted with dichloromethane (200 mL). The organic extracts were washed by water and brine and dried over sodium sulphate. After filtration and evaporation in vacuo there was obtained a white solid of m.p. 169 to 171 °C.

### Example 61

### (3,4-Dihydroxy-2-nitro-phenyl)-phenyl-methanone (compound 5, Table 1)

A stirred suspension of the methyl ether from above (3.53 g, 12.93 mmol) in a mixture of 48% hydrobromic acid (30 mL) and 30% hydrogen bromide in acetic acid (30 mL) was heated at 125 °C for one hour and then allowed to cool to room temperature. The reaction mixture was poured onto ice-water (300 mL) and the resulting yellow/orange precipitate was filtered off and washed with a little water. Recrystallisation from a dichloromethane/petroleum ether mixture afforded orange crystals of m.p. 156 to 157 °C.

### Examples 62-67

By the application of the above described technique and related procedures known to those skilled in the art and using the appropriate methyl ethers, the following compounds were prepared:
1-(3,4-Dihydroxy-2-nitro-phenyl)-2-phenyl ethanone (compound 24, Table 1)
1-(3,4-Dihydroxy-2-nitro-phenyl)-3-phenyl-propan-1-one (compound 14, Table 1)
1-(3,4-Dihydroxy-2-nitro-phenyl)-4-phenyl-butan-1-one (compound 15, Table 1)
1-(3,4-Dihydroxy-2-nitro-phenyl)-5-phenyl-pentan-1-one
1 -(3,4-Dihydroxy-2-nitro-phenyl)-6-phenyl-hexan-1-one
1-(3,4-Dihydroxy-2-nitro-phenyl)-pentan-1-one (compound 17, Table 1).

## Claims

1. A compound of formula I: 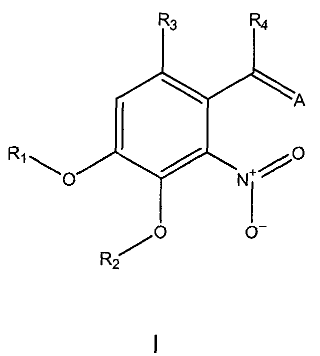 where R₁ and R₂ are the same or different and signify hydrogen, optionally substituted lower alkanoyl or aroyl, optionally substituted lower alkoxycarbonyl, or optionally substituted lower alkylcarbamoyl; R₃ signifies hydrogen or optionally substituted alkanoyl or aroyl group; R₄ signifies optionally substituted saturated or partially unsaturated lower alkyl or aryl group, or taken together with R₃ signifies an optionally substituted saturated or partially unsaturated carbocyclic ring; A signifies oxygen or NR₅ group, where R₅ signifies NHR₆ where R₆ signifies optionally substituted lower alkyl or aryl group, or OR₇ group where R₇ signifies hydrogen, lower alkyl or lower alkanoyl, or A signifies an optionally substituted alkylidene when R₄ signifies OR₈ group where R₈ signifies optionally substituted lower alkanoyl or aroyl group, and pharmaceutically acceptable salts thereof.

2. A compound according to claim 1, wherein R₄ is substituted with at least one aryl or heterocycloalkyl group.

3. A compound according to claim 1, comprising: 6,7-dihydroxy-8-nitro-3,4-dihydro-2H-naphthalen-1-one; 5,6-dihydroxy-7-nitro-indan-1-one; 2-(3,4-dimethoxy-benzylidene)-6,7-dihydroxy-8-nitro-3,4-dihydro-2H-naphthalen-1-one; (3,4-dihydroxy-2-nitro-phenyl)-phenyl-methanone; 5,6-dihydroxy-7-nitro-indan-1-one oxime; 2-(3,4-Dimethoxy-benzylidene)-5,6-dihydroxy-7-nitro-indan-1-one; 2-(4-dimethylamino-benzylidene)-5,6-dihydroxy-7-nitro-indan-1-one; 2-(4-dimethylamino-benzylidene)-6,7-dihydroxy-8-nitro-3,4-dihydro-2H-naphthalen-1-one; 5,6-dihydroxy-2-(4-hydroxy-3-methoxy-5-nitro-benzylidene)-7-nitro-indan-1-one; 6,7-dihydroxy-2-(4-hydroxy-3-methoxy-5-nitro-benzylidene)-8-nitro-3,4-dihydro-2H-naphthalen-1-one; 2-(3,4-dihydroxy-benzylidene)-5,6-dihydroxy-7-nitro-indan-1-one; 2-(3,4-dihydroxy-benzylidene)-6,7-dihydroxy-8-nitro-3,4-dihydro-2H-naphthalen-1-one; 1-(3,4-dihydroxy-2-nitro-phenyl)-3-phenyl-propan-1-one; 1-(3,4-dihydroxy-2-nitro-phenyl)-4-phenyl-butan-1-one; 2-(3,4-dihydroxy-5-nitro-benzylidene)-5,6-dihydroxy-7-nitro-indan-1-one; 1-(3,4-dihydroxy-2-nitro-phenyl)-pentan-1-one; 2,3-dihydroxy-1-nitro-anthraquinone; butyric acid 6-butyryloxy-2-nitro-3-(3-phenyl-propionyl)-phenyl ester; butyric acid 3-benzoyl-6-butyryloxy-2-nitro-phenyl ester; carbonic acid 4-benzoyl-2-ethoxycarbonyloxy-3-nitro-phenyl ester ethyl ester; carbonic acid 2-ethoxycarbonyloxy-3-nitro-4-(3-phenyl-propionyl)-phenyl ester ethyl ester; carbonic acid 4,5-dibenzoyl-2-ethoxycarbonyloxy-3-nitro-phenyl ester ethyl ester; 1-(3,4-dihydroxy-2-nitro-phenyl)-2-phenyl-ethanone; acetic acid 3-acetoxy-7,7-dimethyl-1-nitro-8-oxo-5,6,7,8-tetrahydro-naphthalen-2-yl ester; 1-(3,4-dihydroxy-2-nitro-phenyl)-3-morpholin-4-yl-propan-1-one; 1-(3,4-dihydroxy-2-nitro-phenyl)-3-piperidin-1-yl-propan-1-one; 1-(3,4-dihydroxy-2-nitro-phenyl)-3-pyrrolidin-1-yl-propan-1-one; 5,6-dihydroxy-2-morpholin-4-ylmethyl-7-nitro-indan-1-one; 1-[3-(3,4-dihydroxy-2-nitro-phenyl)-3-oxo-propyl]-piperidine-3-carboxylic acid diethylamide; 1-(3,4-dihydroxy-2-nitro-phenyl)-3-(3-methyl-piperidin-1-yl)-propan-1-one; 1-(3,4-dihydroxy-2-nitro-phenyl)-3-(4-methyl-piperidin-1-yl)-propan-1-one; 1-(3,4-dihydroxy-2-nitro-phenyl)-3-(octahydro-quinolin-1-yl)-propan-1-one; 1-(3,4-dihydroxy-2-nitro-phenyl)-3-(3,5-dimethyl-piperidin-1-yl)-propan-1-one; 3-(4-benzyl-piperidin-1-yl)-1-(3,4-dihydroxy-2-nitro-phenyl)-propan-1-one; 1 -(3,4-dihydroxy-2-nitro-phenyl)-3-[4-(4-methoxy-phenyl)-piperazin-1-yl]-propan-1-one; 1-(3,4-dihydroxy-2-nitro-phenyl)-3-[4-(3-trifluoromethyl-phenyl)-piperazin-1-yl)-propan-1-one; 1-(3,4-dihydroxy-2-nitro-phenyl)-3-(4-propyl-piperazin-1-yl)-propan-1-one; acetic acid 6-acetoxy-2-nitro-3-(3-phenyl-acryloyl)-phenyl ester1-(3,4-Dihydroxy-2-nitro-phenyl)-3-phenyl-propenone; 1-(3,4-dihydroxy-2-nitro-phenyl)-2-morpholin-4-yl-ethanone; 5,6-dihydroxy-7-nitro-2-[4-(3-trifluoromethyl-phenyl)-piperazin-1-ylmethyl]-indan-1-one; 5,6-dihydroxy-7-nitro-2-(4-phenyl-piperazin-1-ylmethyl)-indan-1-one; acetic acid 6-acetoxy-2-nitro-3-phenylacetyl-phenyl ester; acetic acid 2-acetoxy-4-(1-acetoxy-2-phenyl-vinyl)-3-nitro-phenyl ester; butyric acid 6-butyryloxy-2-nitro-3-phenylacetyl-phenyl ester; carbonic acid 2-ethoxycarbonyloxy-3-nitro-4-phenylacetyl-phenyl ester ethyl ester; acetic acid 6-acetoxy-2-nitro-3-(4-phenyl-butyryl)-phenyl ester; butyric acid 6-butyryloxy-2-nitro-3-(4-phenyl-butyryl)-phenyl ester or carbonic acid 2-ethoxycarbonyloxy-3-nitro-4-(4-phenyl-butyryl)-phenyl ester ethyl ester.

4. A method of treating a subject afflicted by some central and peripheral nervous system disorders, where a reduction in the *O*-methylation of catecholamines may be of therapeutical benefit, such as mood disorders, Parkinson's disease and parkinsonian disorders, gastrointestinal disturbances, edema formation states and hypertension, which comprises administering to the subject an amount of a compound according to claim 1, 2 or 3 effective to treat said diseases in the subject.

5. A pharmaceutical composition comprising a therapeutically effective amount of a compound according to claim 1, 2 or 3, in combination with a pharmaceutically acceptable carrier.

6. The use of a compound according to claim 1, 2 or 3, in the manufacture of a medication for treating a subject afflicted by central or peripheral nervous system disorders.

7. The use of a compound according to claim 1, 2 or 3, in the manufacture of a medication for treating mood disorders, Parkinson's disease and parkinsonian disorders, gastrointestinal disturbances, edema formation states and hypertension.

8. The use of a compound according to claim 1, 2 or 3, in therapy.

9. The use of a compound according to claim 1, 2 or 3, in the manufacture of a medicament for use as a COMT inhibitor.
